# EUROPEAN PATENT APPLICATION

(11) **EP 3 466 298 A1**
(43) Date of publication of application: **10.04.2019**
(21) Application number: 17806977.9
(22) Date of filing: 30.05.2017
(51) Int. Cl.: A45D 34/00, A45D 40/00, C08L 75/04, C08G 18/08, C08G 18/42, A61Q 1/00, A61Q 19/00

(54) **HYBRID TYPE FOAM AND COSMETIC KIT USING SAME**

(30) Priority: 30.05.2016 KR 20160066650
(71) Applicant: Cosmax, Inc., Hwaseong-si, Gyeonggi-do 18622 (KR)
(72) Inventor: KIM, Sung Yong, Anyang-si Gyeonggi-do 14000 (KR); HWANG, Chul Hyun, Daejeon 34049 (KR); YOO, Kweon Jong, Suwon-si Gyeonggi-do 16332 (KR); PARK, Myeong Sam, Seoul 04987 (KR); PARK, Chun Ho, Yongin-si Gyeonggi-do 17081 (KR)
(74) Representative: Lavoix
(86) International application number: PCT/KR2017/005631
(87) International publication number: WO 2017/209484

(57) **Abstract**

The present application relates to a hybrid foam including a hybrid copolymer of polyether polyol and polyester polyol, and to a cosmetic kit including the hybrid foam.

## Description

### TECHNICAL FIELD

The present disclosure relates to a hybrid foam for use in impregnating a cosmetic composition, and a cosmetic kit including the hybrid foam. More particularly, the present disclosure relates to a hybrid foam, which includes a hybrid copolymer of a polyether polyol and a polyester polyol, for use in impregnating a cosmetic composition, and a cosmetic kit including the hybrid foam.

### BACKGROUND ART

In general, a cushion compact is a makeup base product for beautifying the face with a natural beige color, and cosmetic contents thereof are usually in a solution preparation (liquids). Such a cushion compact usually contains an impregnant that is impregnated with cosmetics, and is prepared in a double container form including a refillable inner container and an outer container, wherein the inner container includes an impregnant impregnated with cosmetics and the outer container is able to accommodate the refillable inner container by combining therewith.

An impregnant conventionally used in cosmetics may usually include a polyurethane foam. The polyurethane foam may be classified into a polyether polyol foam and a polyester polyol, depending on a type of a polyol used in polymerization.

According to a type of a polyhydroxy compound used in the formation of the polyurethane foam, a polyether polyol foam may be distinguished from a polyester polyol foam. Regarding advantages and disadvantages of each foam, the polyether polyol foam is excellent in hydrolysis resistance, elasticity, and cost, but has weak oil resistance, whereas the polyester polyol foam can easily adjust pores thereof and is excellent in abrasion resistance and chemical resistance, but is also like to undergo hydrolysis and breakage when stored at a high temperature for a long period of time.

Korean Patent No. 10-1560812 discloses a cosmetic container including an impregnant, which is formed of a polycaprolactone polyol foam, and a silver glass antimicrobial component.

However, in consideration of a foam for use in impregnating a cosmetic composition, it is still necessary to develop a new foam material that can complement the disadvantages of the polyether polyol foam and the polyester polyol foam.

### DESCRIPTION OF EMBODIMENTS

### TECHNICAL PROBLEM

Provided are a hybrid foam for use in impregnating a cosmetic composition, the hybrid foam including a hybrid copolymer of a polyether polyol and a polyester polyol, and a cosmetic kit including the hybrid foam.

However, the technical problems to be solved by the present disclosure are not limited to the above-mentioned problems, and other problems not mentioned herein may be clearly understood by those of ordinary skill in the art from the following description.

### SOLUTION TO PROBLEM

According to an aspect of the present disclosure, there is provided a hybrid foam for use in impregnating a cosmetic composition, the hybrid foam including a hybrid copolymer of a polyether polyol and a polyester polyol.

According to another aspect of the present disclosure, there is provided a cosmetic kit including: a spreading puff; a cosmetic composition; and a hybrid foam including a hybrid copolymer of a polyether polyol and a polyester polyol, wherein the hybrid foam is impregnated with the cosmetic composition.

### ADVANTAGEOUS EFFECTS OF DISCLOSURE

In one or more embodiments of the present disclosure, a hybrid foam for use in impregnating a cosmetic composition is not formed by simple mixing of a polyether polyol and a polyester polyol, but includes a hybrid copolymer formed by copolymerization which forms ether-ester chemical bonds between a polyether polyol and a polyester polyol. Thus, due to the chemical bonds formed by copolymerization, the hybrid foam includes a hybrid copolymer having a single skeleton structure, thereby compensating disadvantages possessed by that the existing polyether polyol foam and the existing polyester polyol foam each had. In this regard, the present disclosure may serve as an improved foam for use in impregnating a cosmetic composition. In detail, a foam prepared by simple mixing or blending of a polyether polyol and a polyester polyol may undergo a phase separation, and as a result, the foam may be in a non-uniform state. In this regard, the present disclosure retains the disadvantages that a polyether polyol foam and a polyester polyol foam each have, so that it is difficult to obtain an improved foam. However, in comparison with a conventional urethane foam (e.g., a polyether polyol foam or a polyester polyol foam), since the hybrid foam of the present disclosure simultaneously retains an ether group and an ester groups in a copolymer form, optimum synergistic effect may be produced by providing stabilization at the interface between the ether group and the ester group and by eliminating the interference effect. That is, in one or more embodiments of the present disclosure, excellent physical properties possessed by polyesters and advantages of flexibility and hydrolysis resistance that are possessed by polyethers may be realized. Moreover, the balance between lipophilicity and hydrophilicity may be controlled by a combination of two materials, thereby broadening the gradient degree of supportability depending on the characteristics of the cosmetic composition used herein. Therefore, in one or more embodiments of the present disclosure, various properties may be realized, and when the cosmetic composition is impregnated, the liquid stability at the interface and in the bulk phase may be secured. In addition, in one or more embodiments of the present disclosure, regardless of the degree of hydrophilicity or lipophilicity, which differs according to ingredients of the cosmetic composition that is impregnated in the hybrid foam, various cosmetic compositions may be stably impregnated stably. Therefore, the development of different types of foam according to types or properties of the cosmetic composition is not needed. In particular, the hybrid foam according to one or more embodiments of the present disclosure may control the degree of water-solubility and fat-solubility properties upon changes in polarity depending on the type of element and the type and structure of the bonds. Accordingly, various types of the cosmetic composition used together with the present disclosure may be selected.

In one or more embodiments of the present disclosure, the hybrid foam has excellent chemical resistance, hydrolysis resistance, and durability, compared to the conventional polyurethane foam. In detail, a hybrid foam including a hybrid copolymer of a polyether polyol and a polyester polyol that have excellent hydrolysis properties may be provided. In this regard, problems occurring when only a polyester polyol foam is used, such as problems of hydrolysis of a mash skeleton of an urethane foam by oil-phase ingredients and water-phase ingredients that constitute the cosmetic composition, may be solved. In addition, in one or more embodiments of the present disclosure, a hybrid foam including a hybrid copolymer of a polyether polyol and a polyester polyol that have excellent durability may be provided. In this regard, problems occurring when only a polyether polyol foam was used, such as problems of durability and stability of a foam may be solved. In detail, when a user applies pressure repeatedly and continuously to the foam by using a spread applicator of a cosmetic composition, such as a puff, the foam may be worn and the strength there of may be lowered, resulting in the problems of durability and stability.

In one or more embodiments of the present disclosure, when the hybrid foam including the hybrid copolymer of the polyether polyol and the polyester polyol is used for impregnating a cosmetic composition, the stability of impregnating contents may be secured without separation of the formulations over time.

### BRIEF DESCRIPTION OF DRAWINGS

FIG. 1 is a photograph of a hybrid foam prepared according to an embodiment of the present disclosure.
FIG. 2 is a photograph of a hybrid foam impregnated with a cosmetic composition that is prepared according to an embodiment of the present disclosure.
FIG. 3 is an optical microscopic photograph of a hybrid foam prepared according to an embodiment of the present disclosure.

### BEST MODE

Hereinafter, exemplary embodiments of the present invention will be described in detail with reference to the accompanying drawings so as to be easily carried out by those skilled in the art. However, it should be noted that the present invention is not limited to the following exemplary embodiments and may be implemented in various forms.

Throughout the specification, it will be understood that when a part is referred to as being "linked" with another part, the part can be "directly linked" or "electrically linked" with the other part. That is, for example, intervening elements may be present thereon.

Throughout the specification, it will be understood that when an element is referred to as being "on" another element, the element can be directly or indirectly adjacent to the other element or intervening elements may be present thereon.

Throughout the present specification, when a portion "includes" a component, it means that the portion does not exclude other components but may include other components unless otherwise there is a description to be opposite to this. The terms of degree such as "about" and "substantially" used throughout the present specification are used as the meaning of the presented numerical value or close to the presented numerical value when an intrinsic allowable error of the manufacture and material is presented, and they are used in order to prevent the unscrupulous infringer from exploiting the disclosure having an accurate or absolute numerical value presented in order to facilitate understanding of the present invention. The terms "step of -ing" or "step of -" used throughout the present specification does not mean the "step for -".

Throughput the present specification, the term "any combination thereof" included in the expression of Markush form means one or more mixtures or combinations selected from the group consisting of the components described in the expression of Markush form, and thus it includes one or more selected from the group consisting of the components.

Throughout the specification, the expression "A and/or B" is construed as being "A or B, or both A and B".

The terms "alkyl group" and "alkylene group" as used throughout the specification refer to a linear or branched alkyl group and a linear or branched alkylene group, respectively, each of which has 1 to 24 carbon atoms, 1 to 20 carbon atoms, 1 to 18 carbon atoms, 1 to 15 carbon atoms, 1 to 12 carbon atoms, 1 to 10 carbon atoms, 1 to 8 carbon atoms, 1 to 6carbon atoms, 1 to 5 carbon atoms, or 1 to 3 carbon atoms. When the alkyl group or the alkylene group is substituted with an alkyl group, it is also used interchangeable with "a branched alkyl group" or "a branched alkylene group". The substituent which may be substituted on the alkyl group or the alkylene group may include at least one selected from the group consisting of halogens (e.g., F, CI, Br, or I), halo-alkyl groups (e.g., CC1₃ or CF₃), alkoxy groups, alkylthio groups, hydroxy groups, carboxyl groups (-C(O)-OH), alkyloxycarbonyl groups (-C(O)-O-R), alkylcarbonyloxy groups (-O-C(O)-R), amino groups (-NH₂), carbamoyl groups (-C(O)-NHR), urea groups (-NH-C(O)-NHR-), and thiol groups (-SH), but embodiments of the present disclosure are not limited thereto. Furthermore, atoms among the above-described alkyl group, an alkyl group having at least two carbon atoms may include at least one carbon-carbon double bond or at least one carbon-carbon triple bond, but embodiments of the present disclosure are not limited thereto. Examples of the alkyl group may include methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosanyl, or any possible isomer thereof, but embodiments of the present disclosure are not limited thereto. For example, the alkyl group used herein may be selected from: a C₁-C₁₀ alkyl group, such as a methyl group, an ethyl group, a propyl group, a butyl group, a pentyl group, a hexyl group, a heptyl group, an octyl group, a nonoyl group, or a decyl group; a C₁-C₆ alkyl group, such as a methyl group, an ethyl group, a propyl group, a butyl group, a pentyl group, or a hexyl group; or a C1-C4 alkyl group, such as a methyl group, an ethyl group, an i-propyl group, an n-propyl group, a t-butyl group, an s-butyl group, or an n-butyl group, but embodiments of the present disclosure are not limited thereto.

The term "halogen" or "halo" as used throughout the specification refers to a group including, as a functional group, a halogen atom belonging to Group 17 of the periodic table of the elements, and examples thereof may include chlorine, bromine, fluorine, or iodine, but embodiments of the present disclosure are not limited thereto.

The term "hybrid foam" as used throughout the specification refers to a foam prepared by processing polyurethane in the form of a foam by a reaction between a hybrid copolymer of a polyether polyol and a polyester polyol of the present disclosure and a compound including an isocyanate (-NCO) group.

The term "polyether polyol foam" as used throughout the specification refers to a foam prepared by processing polyurethane in the form of a foam by a reaction between a polyether polyol and a compound including an isocyanate (-NCO) group.

The term "polyester polyol foam" as used throughout the specification refers to a foam prepared by processing polyurethane in the form of a foam by a reaction between a polyester polyol and a compound including an isocyanate (-NCO) group.

Hereinafter, embodiments will be described in detail by explaining exemplary embodiments with reference to the accompanying drawings. However, the present disclosure should not be construed as limited to the exemplary embodiments and drawings set forth herein.

An aspect of the present disclosure provides a hybrid foam for use in impregnating a cosmetic composition, the hybrid foam including a hybrid copolymer of a polyether polyol and a polyester polyol.

In one embodiment, the hybrid copolymer may include a copolymer formed by copolymerizing X and Y, wherein X indicates an ether structure-containing polyol or a monomer or oligomer for forming the polyether polyol, and Y indicates an ester structure-containing polyol or a monomer or oligomer for forming the polyester polyol.

In one embodiment, the hybrid copolymer may be represented by (XY)ₙX, wherein X indicates the polyether polyol, Y indicates the polyester polyol, and n indicates a number in a range of 1 to 200. Here, n may vary according to X and Y to be selected. However, embodiments of the present disclosure are not limited thereto. For example, the hybrid copolymer represented by (XY)ₙX may be formed by copolymerizing X and Y at a ratio of n+1, wherein X indicates an ether structure-containing polyol or a monomer or oligomer for forming the polyether polyol, and Y indicates an ester structure-containing polyol or a monomer or oligomer for forming the polyester polyol. However, embodiments of the present disclosure are not limited thereto. For example, n may be a number in a range of about 1 to about 200, for example, about 1 to about 200, about 1 to about 180, about 1 to about 160, about 1 to about 150, about 1 to about 140, about 1 to about 120, about 1 to about 100, about 1 to about 90, about 1 to about 80, about 1 to about 70, about 1 to about 60, about 1 to about 50, about 1 to about 40, about 1 to about 30, about 1 to about 20, about 1 to about 15, about 1 to about 10, about 1 to about 9, about 1 to about 8, about 1 to about 7, about 1 to about 6, about 1 to about 5, about 1 to about 4, about 1 to about 3, about 1 to about 2, about 3 to about 200, about 3 to about 180, about 3 to about 160, about 3 to about 150, about 3 to about 140, about 3 to about 120, about 3 to about 100, about 3 to about 50, about 3 to about 40, about 3 to about 30, about 3 to about 20, about 3 to about 15, about 3 to about 10, about 3 to about 9, about 3 to about 8, about 3 to about 7, about 3 to about 6, about 3 to about 5, about 3 to about 4, about 5 to about 200, about 5 to about 180, about 5 to about 160, about 5 to about 150, about 5 to about 140, about 5 to about 120, about 5 to about 100, about 5 to about 90, about 5 to about 80, about 5 to about 70, about 5 to about 60, about 5 to about 50, about 5 to about 40, about 5 to about 30, about 5 to about 20, about 5 to about 15, about 5 to about 10, about 5 to about 9, about 5 to about 8, about 5 to about 7, about 5 to about 6, about 7 to about 200, about 7 to about 180, about 7 to about 160, about 7 to about 150, about 7 to about 140, about 7 to about 120, about 7 to about 100, about 7 to about 90, about 7 to about 80, about 7 to about 70, about 7 to about 60, about 7 to about 50, about 7 to about 40, about 7 to about 30, about 7 to about 20, about 7 to about 15, about 7 to about 10, about 7 to about 9, about 7 to about 8, about 10 to about 200, about 10 to about 180, about 10 to about 160, about 10 to about 150, about 10 to about 140, about 10 to about 120, about 10 to about 100, about 10 to about 90, about 10 to about 80, about 10 to about 70, about 10 to about 60, about 10 to about 50, about 10 to about 40, about 10 to about 30, or about 10 to about 20, but embodiments of the present disclosure are not limited thereto. In one or more embodiments, when the hybrid copolymer is prepared within the range of the above-mentioned n value, the elasticity and commercial processability suitable for carrying the cosmetic composition may be possibly secured.

In one embodiment, the hybrid copolymer represented by (XY)ₙX may be represented by Formula 1. In Formula 1, R may be an alkylene group or an ether oligomer, and R' may be an alkylene group, or an ester oligomer, wherein the alkylene, the ether oligomer, and the ester oligomer may each include a saturated or unsaturated carbon bond and may each include a linear or branched structure, but embodiments of the present disclosure are not limited thereto:

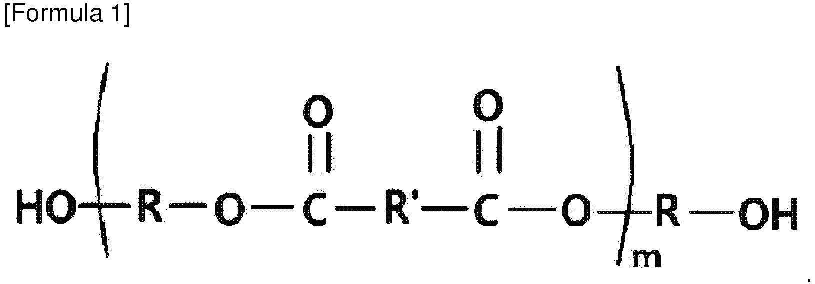

In Formula 1, m may be a number in a range o about 1 to about 200, for example, about 1 to about 200, about 1 to about 180, about 1 to about 160, about 1 to about 150, about 1 to about 140, about 1 to about 120, about 1 to about 100, about 1 to about 90, about 1 to about 80, about 1 to about 70, about 1 to about 60, about 1 to about 50, about 1 to about 40, about 1 to about 30, about 1 to about 20, about 1 to about 15, about 1 to about 10, about 1 to about 9, about 1 to about 8, about 1 to about 7, about 1 to about 6, about 1 to about 5, about 1 to about 4, about 1 to about 3, about 1 to about 2, about 3 to about 200, about 3 to about 180, about 3 to about 160, about 3 to about 150, about 3 to about 140, about 3 to about 120, about 3 to about 100, about 3 to about 50, about 3 to about 40, about 3 to about 30, about 3 to about 20, about 3 to about 15, about 3 to about 10, about 3 to about 9, about 3 to about 8, about 3 to about 7, about 3 to about 6, about 3 to about 5, about 3 to about 4, about 5 to about 200, about 5 to about 180, about 5 to about 160, about 5 to about 150, about 5 to about 140, about 5 to about 120, about 5 to about 100, about 5 to about 90, about 5 to about 80, about 5 to about 70, about 5 to about 60, about 5 to about 50, about 5 to about 40, about 5 to about 30, about 5 to about 20, about 5 to about 15, about 5 to about 10, about 5 to about 9, about 5 to about 8, about 5 to about 7, about 5 to about 6, about 7 to about 200, about 7 to about 180, about 7 to about 160, about 7 to about 150, about 7 to about 140, about 7 to about 120, about 7 to about 100, about 7 to about 90, about 7 to about 80, about 7 to about 70, about 7 to about 60, about 7 to about 50, about 7 to about 40, about 7 to about 30, about 7 to about 20, about 7 to about 15, about 7 to about 10, about 7 to about 9, about 7 to about 8, about 10 to about 200, about 10 to about 180, about 10 to about 160, about 10 to about 150, about 10 to about 140, about 10 to about 120, about 10 to about 100, about 10 to about 90, about 10 to about 80, about 10 to about 70, about 10 to about 60, about 10 to about 50, about 10 to about 40, about 10 to about 30, or about 10 to about 20, but embodiments of the present disclosure are not limited thereto. In one or more embodiments, when the hybrid copolymer is prepared within the range of m described above, the elasticity and commercialized processability suitable for carrying the cosmetic composition may be secured.

In one embodiment, R and R' in Formula 1 may each independently include carbon atoms in the number ranging from about 1 to about 450, about 1 to about 400, about 1 to about 350, about 1 to about 300, about 1 to about 250, about 1 to about 200, about 1 to about 150, about 1 to about 100, about 1 to about 80, about 1 to about 60, about 1 to about 40, about 1 to about 20, about 1 to about 15, about 1 to about 10, about 1 to about 8, about 1 to about 6, about 1 to about 4, or about 1 to about 2, about 2 to about 450, about 2 to about 400, about 2 to about 350, about 2 to about 300, about 2 to about 250, about 2 to about 200, about 2 to about 150, about 2 to about 100, about 2 to about 80, about 2 to about 60, about 2 to about 40, about 2 to about 20, about 2 to about 15, about 2 to about 10, about 2 to about 8, about 2 to about 6, about 2 to about 4, about 3 to about 450, about 3 to about 400, about 3 to about 350, about 3 to about 300, about 3 to about 250, about 3 to about 200, about 3 to about 150, about 3 to about 100, about 3 to about 80, about 3 to about 60, about 3 to about 40, about 3 to about 20, about 3 to about 15, about 3 to about 10, about 3 to about 8, about 3 to about 6, about 3 to about 4, about 4 to about 450, about 4 to about 400, about 4 to about 350, about 4 to about 300, about 4 to about 250, about 4 to about 200, about 4 to about 150, about 4 to about 100, about 4 to about 80, about 4 to about 60, about 4 to about 40, about 4 to about 20, about 4 to about 15, about 4 to about 10, about 4 to about 8, about 4 to about 6, about 5 to about 450, about 5 to about 400, about 5 to about 350, about 5 to about 300, about 5 to about 250, about 5 to about 200, about 5 to about 150, about 5 to about 100, about 5 to about 80, about 5 to about 60, about 5 to about 40, about 5 to about 20, about 5 to about 15, about 5 to about 10, about 5 to about 8, about 5 to about 6, about 10 to about 450, about 10 to about 400, about 10 to about 350, about 10 to about 300, about 10 to about 250, about 10 to about 200, about 10 to about 150, about 10 to about 100, about 10 to about 80, about 10 to about 60, about 10 to about 40, about 10 to about 20, about 10 to about 15, about 20 to about 450, about 20 to about 400, about 20 to about 350, about 20 to about 300, about 20 to about 250, about 20 to about 200, about 20 to about 150, about 20 to about 100, about 20 to about 80, about 20 to about 60, about 20 to about 40, about 30 to about 450, about 30 to about 400, about 30 to about 350, about 30 to about 300, about 30 to about 250, about 30 to about 200, about 30 to about 150, about 30 to about 100, about 30 to about 80, about 30 to about 60, about 30 to about 40, about 40 to about 450, about 40 to about 400, about 40 to about 350, about 40 to about 300, about 40 to about 250, about 40 to about 200, about 40 to about 150, about 40 to about 100, about 40 to about 80, about 40 to about 60, about 50 to about 450, about 50 to about 400, about 50 to about 350, about 50 to about 300, about 50 to about 250, about 50 to about 200, about 50 to about 150, about 50 to about 100, about 50 to about 80, about 50 to about 60, about 100 to about 450, about 100 to about 400, about 100 to about 350, about 100 to about 300, about 100 to about 250, about 100 to about 200, about 100 to about 150, about 200 to about 450, about 200 to about 400, about 200 to about 350, about 200 to about 300, about 200 to about 250, about 300 to about 450, about 300 to about 400, or about 300 to about 350, but embodiments of the present disclosure are not limited thereto. When the number of carbon atoms in R or R' in Formula 1 is greater than 450, due to a large molecular weight of the hybrid copolymer, problems in stability and processability may occur while unnecessary side reactions may be involved in the synthesis of the hybrid copolymer.

In one embodiment, the hybrid copolymer may further include a known polyether polyol or a known polyester polyol to ensure the compatibility, but embodiments of the present disclosure are not limited thereto.

In one embodiment, the monomer for forming the polyether polyol may be selected from an alkylene oxide, a diol, a triol, or any combination thereof, and the monomer of the polyester polyol may be an organic acid including at least two carboxyl groups, but embodiments of the present disclosure are not limited thereto.

In one embodiment, the monomer for forming the polyether polyol may be selected from: an alkylene oxide selected from etylene oxide and propylene oxide; a dial selected from dipropylene glycol (hereinafter, referred to as "DPG"), mono-ethylene glycol (hereinafter, referred to as "MEG") and propylene glycol (hereinafter, referred to as "PG"); a trial selected from glycerin and trimethylol propane (hereinafter, referred to as "TMP"); and any combination thereof, but embodiments of the present disclosure are not limited thereto.

In one or more embodiments, the polyether polyol may be obtained by addition polymerization between an active hydrogen atom-containing initiator and an alkylene oxide, such as propylene oxide or ethylene oxide, but embodiments of the present disclosure are not limited thereto. The initiator may be usually selected from: propylene glycol, dipropylene glycol, ethylene glycol, diethylene glycol, diethanol amine, bisphenol, and water, which have two functional groups; glycerine, trimethylol propane, triethanol amine, and haxanetriol, which have three functional groups; and any combination thereof, but embodiments of the present disclosure are not limited thereto.

In one embodiment, to enhance the hardness of the polyether polyol, an initiator prepared by copolymerizing a vinyl group-containing monomer, such as a styrene monomer or an acrylonitrile monomer, with the synthesized polyether polyol, or an initiator, such as pentaerythritol, ethylenediamine, toluenediamine, sorbitol, or sucrose, which has at least three functional groups, may be partially used at a ratio in a range of about 1% to about 30%, but embodiments of the present disclosure are not limited thereto.

In one embodiment, the monomer of the polyester polyol may include a multivalent organic acid including at least two carboxyl groups and about 2 to about 20 carbon atoms, and examples of the multivalent organic acid may be a higher fatty acid, a saturated fatty acid, or an unsaturated fatty acid, but embodiments of the present disclosure are not limited thereto. For example, the number of carbons atoms in the multivalent organic acid may be in a range of about 2 to about 20, about 2 to about 18, about 2 to about 16, about 2 to about 14, about 2 to about 12, about 2 to about 10, about 2 to about 8, about 2 to about 6, or about 2 to about 4, but embodiments of the present disclosure are not limited thereto.

In one embodiment, the multivalent organic acid including at least two carboxyl groups may be, for example, selected from adipic acid, oxalic acid, and succinic acid, but embodiments of the present disclosure are not limited thereto.

In one embodiment, the monomer of the polyester polyol may be selected from soybean oil, castor oil, citric acid, palmitic acid, myristic acid, oleic acid, stearic acid, linoleic acid, and linolenic acid, but embodiments of the present disclosure are not limited thereto.

In one embodiment, following the synthesis of the polyether polyol, an organic acid including at least two carboxylic acid, such as adipic acid or fatty acid, may be added thereto to induce esterification via dehydration condensation, so as to synthesize a copolymer in which ether-ester bonds coexist in one polymer. Here, the temperature at which the hybrid copolymer is synthesized via esterification may be room temperature or above room temperature, or may be in a range of about room temperature to about 250°C, about room temperature to about 200°C, about room temperature to about 150°C, about room temperature to about 100°C, about 50°C to about 250°C, about 50°C to about 200°C, about 50°C to about 150°C, about 50°C to about 100°C, about 100°C to about 250°C, about 100°C to about 200°C, about 100°C to about 150°C, about 150°C to about 250°C, about 150°C to about 200°C, or about 200°C to about 250°C, but embodiments of the present disclosure are not limited thereto.

In one embodiment, as polyurethane is formed by a reaction with a compound including an isocyanate group (-NCO) in the presence of a catalyst, a foam stabilizer, a foaming agent, and if needed, other flame retardants, fillers, lubricants, and cross-linking agents, the synthesized hybrid copolymer may form a hybrid foam. The polyurethane may be produced by synthesis between a material with an active hydrogen atom and a material with an isocyanate group (-NCO), but embodiments of the present disclosure are not limited thereto.

In one embodiment, the compound including the isocyanate group may be selected from aromatic, alicyclic, and aliphatic polyisocianates having at least two isocyanate groups, any combination thereof, and modified polyisocyanates obtained by modification performed on the aromatic, alicyclic, and aliphatic polyisocianates, but embodiments of the present disclosure are not limited thereto.

In one embodiment, the compound including the isocyanate group may be selected from: aromatic diisocyanate, such as methylene diphenyl diisocyanate (MDI), toluene diisocyanate (TDI), 2,4- or 2,6-tolylene diisocyanate, xylylene diisocyanate (XDI), paraphenylene diisocyanate, 1,5-naphthalene diisocyanate, or tolidine diisocyanate; aliphatic diisocyanate with an aromatic ring, such as α,α,α',α'-tetramethyl xylylene diisocyanate; aliphatic diisocyanate, such as methylene diisocyanate, propylene diisocyanate, lysine diisocyanate, 2,2,4- or 2,4,4-trimethyl hexamethylene diisocyanate, or 1,6-hexamethylene diisocyanate (HMDI); alicyclic diisocyanate, such as 1,4-cyclohexane diisocyanate, methylcyclohexane diisocyanate (TDI with hydrogen), 1-isocyanate-3-isocyanatemethyl-3,5,5-trimethylcyclohesane (IPDI), 4,4'-dicyclohexylmethane diisocyanate, or isopropylidenedicyclohexyl-4,4'-diisocyanate; and any combination thereof, but embodiments of the present disclosure are not limited thereto.

In one embodiment, the amount of the polyisocyanate compound being used may be in a range of about 30 to about 130, about 40 to about 120, or about 60 to about 100, and more preferably, about 50 to about 110, in terms of an isocyanate index, but embodiments of the present disclosure are not limited thereto. The isocyanate index refers to a numerical value which is about 100 times the number of the isocyanate group(s) with respect to the total of all the active hydrogen-rich water molecules including polyols, cross-linking agents, water, or the like.

In one embodiment, the catalyst may include any catalyst that promotes urethanization. For example, the catalyst may be selected from: tertiary amines, such as triethylenediamine, bis(2-dimethylaminoethyl)ether, and N,N,N',N'-tetramethylhexa methylenediamine; metal salts of carboxylic acids, such as potassium acetate and 2-ethylhexane potassium; organometallic compounds, such as stannous octoate, and dibutyl tin dilaurate; and any combination thereof, but embodiments of the present disclosure are not limited thereto.

In one embodiment, the foam stabilizer may be used without limitation as being commonly used in the production of a polyurethane form, and examples thereof may include a silicon-based foam stabilizer and a fluorine-based foam stabilizer. However, embodiments of the present disclosure are not limited thereto.

In one embodiment, the foam stabilizer may be preferably a silicon-based foam stabilizer, and the silicon content of the foam stabilizer may be in a range of about 10 parts to about 50 parts by weight, more preferably, about 30 parts to about 50 parts by weight, based on 100 parts by weigh of the foam stabilizer. However, embodiments of the present disclosure are not limited thereto. The silicon content is a ratio of a polysiloxane chain to the foam stabilizer, and the remainder is a polyoxyalkylene chain. In addition, the content of an oxyethylene group in the polyoxyalkylene chain may be in a range of about 70 parts to about 100 parts, more preferably, about 90 parts to about 100 parts by weight, based on 100 parts by weight of the polyoxyalkylene chain. However, embodiments of the present disclosure are not limited thereto.

In one embodiment, the foaming agent may be selected from fluorinated hydrocarbon, water, and inert gas, and any combination thereof, and the inert gas may include air, nitrogen gas, and carbon dioxide gas. The amount of the foaming agent being used is not particularly limited, but when water is used, the amount of the foaming agent may be about 10 parts by weight or less or in a range of about 0.5 parts to about 4 parts by weight, based on 100 parts by weight of the polyol mixture. However, embodiments of the present disclosure are not limited thereto.

In one embodiment, an additive may be further added in the process of forming the polyurethane. The additive may be, for example, selected from: a filler, such as potassium carbonate or barium sulfate; a surfactant, such as an emulsifier; an antiaging agent, such as antioxidant or a UV absorber; a flame retardant; a plasticizer; a coloring agent; an antifungal agent; a defoamer; a dispersant; anti-tarnish agent; and any combination thereof, but embodiments of the present disclosure are not limited thereto.

In one embodiment, the amount of the foam stabilizer may be in a range of about 0.01 parts to about 5 parts by weight, more preferably, about 0.1 parts to about 2 pars by weight, based on 100 parts by weight of the polyol (wherein, a cross-linking agent is not included in the polyol), but embodiments of the present disclosure are not limited thereto.

In one embodiment, a dial including dipropylene glycol (DPG) as an initiator may be subjected to addition polymerization with propylene oxide (PO), and accordingly, a polyether polyol having a molecular weight in a range of about 100 to about 8,000 may be primarily formed. Then, an organic acid, such as adipic acid, may be added to the polymerized polyether polyol, and via dehydration condensation, the resulting compound may be esterified, thereby forming a copolymer having a hybrid structure of a polyester polyol and a polyether polyol that are copolymerized. To the copolymer formed therefrom, a pigment, a vulcanizing agent, a foaming agent, other additives, and water may be added and mixed. The mixture may be then stirred with isocyanate to foam, followed by being injected to a mold to solidify. Afterwards, the foam molded as being subjected to a heating process in a heating furnace and to vulcanization may be cut, dried, and surface-treated, thereby preparing a hybrid foam, but embodiments of the present disclosure are not limited thereto.

In one embodiment, the molecular weight of the hybrid copolymer may be in a range of about 100 to about 20,000, about 100 to about 18,000, about 100 to about 16,000, about 100 to about 14,000, about 100 to about 12,000, about 100 to about 10,000, about 100 to about 9,000, about 100 to about 8,000, about 100 to about 7,000, about 100 to about 6,000, about 100 to about 5,000, about 100 to about 4,000, about 100 to about 3,000, about 100 to about 2,000, about 500 to about 20,000, about 500 to about 18,000, about 500 to about 16,000, about 500 to about 14,000, about 500 to about 12,000, about 500 to about 10,000, about 500 to about 9,000, about 500 to about 8,000, about 500 to about 7,000, about 500 to about 6,000, about 500 to about 5,000, about 500 to about 4,000, about 500 to about 3,000, about 500 to about 2,000, about 1,000 to about 20,000, about 1,000 to about 18,000, about 1,000 to about 16,000, about 1,000 to about 14,000, about 1,000 to about 12,000, about 1,000 to about 10,000, about 1,000 to about 9,000, about 1,000 to about 8,000, about 1,000 to about 7,000, about 1,000 to about 6,000, about 1,000 to about 5,000, about 1,000 to about 4,000, about 1,000 to about 3,000, about 1,000 to about 2,000, about 2,000 to about 20,000, about 2,000 to about 18,000, about 2,000 to about 16,000, about 2,000 to about 14,000, about 2,000 to about 12,000, about 2,000 to about 10,000, about 2,000 to about 9,000, about 2,000 to about 8,000, about 2,000 to about 7,000, about 2,000 to about 6,000, about 2,000 to about 5,000, about 2,000 to about 4,000, about 2,000 to about 3,000, about 3,000 to about 20,000, about 3,000 to about 18,000, about 3,000 to about 16,000, about 3,000 to about 14,000, about 3,000 to about 12,000, about 3,000 to about 10,000, about 3,000 to about 9,000, about 3,000 to about 8,000, about 3,000 to about 7,000, about 3,000 to about 6,000, about 3,000 to about 5,000, about 3,000 to about 4,000, about 4,000 to about 20,000, about 4,000 to about 18,000, about 4,000 to about 16,000, about 4,000 to about 14,000, about 4,000 to about 12,000, about 4,000 to about 10,000, about 4,000 to about 9,000, about 4,000 to about 8,000, about 4,000 to about 7,000, about 4,000 to about 6,000, about 4,000 to about 5,000, about 5,000 to about 20,000, about 5,000 to about 18,000, about 5,000 to about 16,000, about 5,000 to about 14,000, about 5,000 to about 12,000, about 5,000 to about 10,000, about 5,000 to about 9,000, about 5,000 to about 8,000, about 5,000 to about 7,000, about 5,000 to about 6,000, about 6,000 to about 20,000, about 6,000 to about 18,000, about 6,000 to about 16,000, about 6,000 to about 14,000, about 6,000 to about 12,000, about 6,000 to about 10,000, about 6,000 to about 9,000, about 6,000 to about 8,000, about 6,000 to about 7,000, about 7,000 to about 20,000, about 7,000 to about 18,000, about 7,000 to about 16,000, about 7,000 to about 14,000, about 7,000 to about 12,000, about 7,000 to about 10,000, about 7,000 to about 9,000, about 7,000 to about 8,000, about 8,000 to about 10,000, about 8,000 to about 9,000, about 2,500 to about 3,500, about 3,500 to about 4,500, about 4,500 to about 5,500, or about 5,500 to about 6,500. When the molecular weight of the hybrid copolymer is less than 1,000, the elasticity of the foam including the hybrid copolymer may be reduced while the hardness thereof may be increased, resulting in a reduced sense of touch. When the molecular weight of the hybrid copolymer is greater than 20,000, the processing of the foam including the hybrid copolymer may be difficult, and the foam may be so soft that the durability of the foam may be reduced.

In one embodiment, the hybrid foam may include open pores, but embodiments of the present disclosure are not limited thereto.

In one embodiment, number of pores per inch (ppi) of the open pores in the hybrid foam may be in a range of about 70 ppi to about 120 ppi, about 70 ppi to about 110 ppi, about 70 ppi to about 100 ppi, about 80 ppi to about 120 ppi, about 80 ppi to about 110 ppi, about 80 ppi to about 100 ppi, about 90 ppi to about 120 ppi, about 90 ppi to about 110 ppi, or about 90 ppi to about 100 ppi, but embodiments of the present disclosure are not limited thereto. When the number of the open pores in the hybrid foam is less than about 70 ppi, the elasticity of the foam may be reduced, resulting in poor usability, and the fluidity of the cosmetic composition may be difficult to control. When the number of the open pores in the hybrid foam is greater than about 120 ppi, the durability and usability of the contents may be degraded.

In one embodiment, the size of the open pores in the hybrid foam may be in a range of about 0.01 mm to about 0.35 mm, about 0.05 mm to about 0.35 mm, about 0.10 mm to about 0.35 mm, about 0.15 mm to about 0.35 mm, about 0.20 mm to about 0.35 mm, about 0.25 mm to about 0.35 mm, or about 0.30 mm to about 0.35 mm, but embodiments of the present disclosure are not limited thereto. When the size of the open pores in the hybrid foam is smaller than the range above, the impregnation of the hybrid foam may be difficult, and in addition, the usage efficiency of hybrid foam may be small compared with the amount of the contents filled therein. When the size of the open pores in the hybrid foam is greater than the range above, the usage efficiency of hybrid foam may be good compared with the amount of the contents filled therein. However, when the pore size is too large, the stability of the contents of the hybrid foam is not good so that the strength required for collecting the cosmetic composition is small, resulting in easy discharge of the contents upon a weak impact applied thereto. That is, there may be a problem with poor discharge control ability of the cosmetic composition.

In one embodiment, by using an Asker hardness tester type F, the hardness of the hybrid foam may be measured in a range of about 10 to about 70, about 10 to about 60, about 10 to about 50, about 10 to about 40, about 10 to about 30, or about 10 to about 20, but embodiments of the present disclosure are not limited thereto.

Another aspect of the present invention provides a cosmetic kit including: a cosmetic composition; and a hybrid foam including a hybrid copolymer of a polyether polyol and a polyester polyol, wherein the hybrid foam is impregnated with the cosmetic composition.

The hybrid foam may be the same as the one used according to the first aspect and one or more embodiments of the present disclosure. Thus, all the descriptions of the hybrid foam of the first aspect and one or more embodiments of the present disclosure may be applied to the hybrid foam of the another aspect.

In one embodiment, the cosmetic kit may further include a spreading applicator. The spreading applicator may include, without limitation, any product available for use in spreading the cosmetic composition impregnated in the hybrid foam, and examples thereof may include a brush, a puff, a sponge, a roller, or a stick. However, embodiments of the present disclosure are not limited thereto.

In one embodiment, the cosmetic kit may further include a container for accommodating the hybrid foam.

In one embodiment, the cosmetic kit may further include an inner container for accommodating the hybrid foam and an outer container containing the inner container.

In one embodiment, the cosmetic kit may be a compact type kit including: a spreading applicator; an impregnant impregnated with the cosmetic composition; an inner container including the impregnant; and an outer container accommodating the inner container, wherein the impregnant is the hybrid foam including the hybrid copolymer of the polyether polyol and the polyester polyol of the aspect of the present disclosure.

In one embodiment, the hybrid copolymer may include a copolymer formed by copolymerizing X and Y, wherein X indicates an ether structure-containing polyol or a monomer or oligomer for forming the polyether polyol, and Y indicates an ester structure-containing polyol or a monomer or oligomer for forming the polyester polyol.

In one embodiment, the hybrid copolymer may be represented by (XY)ₙX, wherein X indicates the polyether polyol, Y indicates the polyester polyol, and n indicates a number in a range of 1 to 200. Here, n may vary according to X and Y to be selected, but embodiments of the present disclosure are not limited thereto. For example, the hybrid copolymer represented by (XY)ₙX may be formed by copolymerizing X and Y at a ratio of n+1, wherein X indicates an ether structure-containing polyol or a monomer or oligomer for forming the polyether polyol, and Y indicates an ester structure-containing polyol or a monomer or oligomer for forming the polyester polyol. However, embodiments of the present disclosure are not limited thereto. For example, n may be a number in a range of about 1 to about 200, for example, about 1 to about 200, about 1 to about 180, about 1 to about 160, about 1 to about 150, about 1 to about 140, about 1 to about 120, about 1 to about 100, about 1 to about 90, about 1 to about 80, about 1 to about 70, about 1 to about 60, about 1 to about 50, about 1 to about 40, about 1 to about 30, about 1 to about 20, about 1 to about 15, about 1 to about 10, about 1 to about 9, about 1 to about 8, about 1 to about 7, about 1 to about 6, about 1 to about 5, about 1 to about 4, about 1 to about 3, about 1 to about 2, about 3 to about 200, about 3 to about 180, about 3 to about 160, about 3 to about 150, about 3 to about 140, about 3 to about 120, about 3 to about 100, about 3 to about 50, about 3 to about 40, about 3 to about 30, about 3 to about 20, about 3 to about 15, about 3 to about 10, about 3 to about 9, about 3 to about 8, about 3 to about 7, about 3 to about 6, about 3 to about 5, about 3 to about 4, about 5 to about 200, about 5 to about 180, about 5 to about 160, about 5 to about 150, about 5 to about 140, about 5 to about 120, about 5 to about 100, about 5 to about 90, about 5 to about 80, about 5 to about 70, about 5 to about 60, about 5 to about 50, about 5 to about 40, about 5 to about 30, about 5 to about 20, about 5 to about 15, about 5 to about 10, about 5 to about 9, about 5 to about 8, about 5 to about 7, about 5 to about 6, about 7 to about 200, about 7 to about 180, about 7 to about 160, about 7 to about 150, about 7 to about 140, about 7 to about 120, about 7 to about 100, about 7 to about 90, about 7 to about 80, about 7 to about 70, about 7 to about 60, about 7 to about 50, about 7 to about 40, about 7 to about 30, about 7 to about 20, about 7 to about 15, about 7 to about 10, about 7 to about 9, about 7 to about 8, about 10 to about 200, about 10 to about 180, about 10 to about 160, about 10 to about 150, about 10 to about 140, about 10 to about 120, about 10 to about 100, about 10 to about 90, about 10 to about 80, about 10 to about 70, about 10 to about 60, about 10 to about 50, about 10 to about 40, about 10 to about 30, or about 10 to about 20, but embodiments of the present disclosure are not limited thereto. In one or more embodiments, when the hybrid copolymer is prepared within the range of the above-mentioned n value, the elasticity and commercial processability suitable for carrying the cosmetic composition may be possibly secured.

In one embodiment, the hybrid copolymer represented by (XY)ₙX may be represented by Formula 1. In Formula 1, R may be an alkylene group or an ether oligomer, and R' may be an alkylene group or an ester oligomer, wherein the alkylene, the ether oligomer, and the ester oligomer may each include a saturated or unsaturated carbon bond and a linear or branched structure, but embodiments of the present disclosure are not limited thereto:

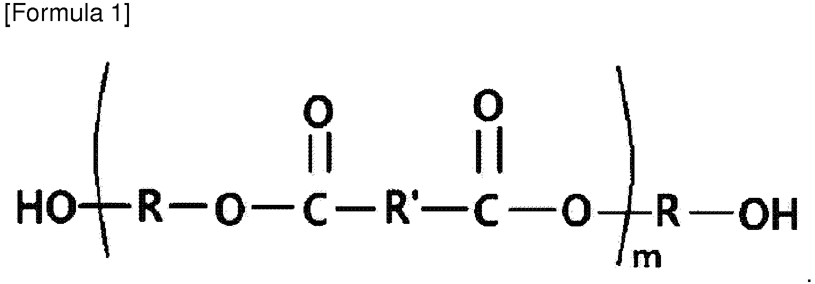

In Formula 1, m may be a number in a range o about 1 to about 200, for example, about 1 to about 200, about 1 to about 180, about 1 to about 160, about 1 to about 150, about 1 to about 140, about 1 to about 120, about 1 to about 100, about 1 to about 90, about 1 to about 80, about 1 to about 70, about 1 to about 60, about 1 to about 50, about 1 to about 40, about 1 to about 30, about 1 to about 20, about 1 to about 15, about 1 to about 10, about 1 to about 9, about 1 to about 8, about 1 to about 7, about 1 to about 6, about 1 to about 5, about 1 to about 4, about 1 to about 3, about 1 to about 2, about 3 to about 200, about 3 to about 180, about 3 to about 160, about 3 to about 150, about 3 to about 140, about 3 to about 120, about 3 to about 100, about 3 to about 50, about 3 to about 40, about 3 to about 30, about 3 to about 20, about 3 to about 15, about 3 to about 10, about 3 to about 9, about 3 to about 8, about 3 to about 7, about 3 to about 6, about 3 to about 5, about 3 to about 4, about 5 to about 200, about 5 to about 180, about 5 to about 160, about 5 to about 150, about 5 to about 140, about 5 to about 120, about 5 to about 100, about 5 to about 90, about 5 to about 80, about 5 to about 70, about 5 to about 60, about 5 to about 50, about 5 to about 40, about 5 to about 30, about 5 to about 20, about 5 to about 15, about 5 to about 10, about 5 to about 9, about 5 to about 8, about 5 to about 7, about 5 to about 6, about 7 to about 200, about 7 to about 180, about 7 to about 160, about 7 to about 150, about 7 to about 140, about 7 to about 120, about 7 to about 100, about 7 to about 90, about 7 to about 80, about 7 to about 70, about 7 to about 60, about 7 to about 50, about 7 to about 40, about 7 to about 30, about 7 to about 20, about 7 to about 15, about 7 to about 10, about 7 to about 9, about 7 to about 8, about 10 to about 200, about 10 to about 180, about 10 to about 160, about 10 to about 150, about 10 to about 140, about 10 to about 120, about 10 to about 100, about 10 to about 90, about 10 to about 80, about 10 to about 70, about 10 to about 60, about 10 to about 50, about 10 to about 40, about 10 to about 30, or about 10 to about 20, but embodiments of the present disclosure are not limited thereto. In one or more embodiment, when the hybrid copolymer is prepared within the range of m described above, the elasticity and commercialized processability suitable for carrying the cosmetic composition may be secured.

In one embodiment, R and R' in Formula 1 may each independently include carbon atoms in the number ranging from about 1 to about 450, about 1 to about 400, about 1 to about 350, about 1 to about 300, about 1 to about 250, about 1 to about 200, about 1 to about 150, about 1 to about 100, about 1 to about 80, about 1 to about 60, about 1 to about 40, about 1 to about 20, about 1 to about 15, about 1 to about 10, about 1 to about 8, about 1 to about 6, about 1 to about 4, or about 1 to about 2, about 2 to about 450, about 2 to about 400, about 2 to about 350, about 2 to about 300, about 2 to about 250, about 2 to about 200, about 2 to about 150, about 2 to about 100, about 2 to about 80, about 2 to about 60, about 2 to about 40, about 2 to about 20, about 2 to about 15, about 2 to about 10, about 2 to about 8, about 2 to about 6, about 2 to about 4, about 3 to about 450, about 3 to about 400, about 3 to about 350, about 3 to about 300, about 3 to about 250, about 3 to about 200, about 3 to about 150, about 3 to about 100, about 3 to about 80, about 3 to about 60, about 3 to about 40, about 3 to about 20, about 3 to about 15, about 3 to about 10, about 3 to about 8, about 3 to about 6, about 3 to about 4, about 4 to about 450, about 4 to about 400, about 4 to about 350, about 4 to about 300, about 4 to about 250, about 4 to about 200, about 4 to about 150, about 4 to about 100, about 4 to about 80, about 4 to about 60, about 4 to about 40, about 4 to about 20, about 4 to about 15, about 4 to about 10, about 4 to about 8, about 4 to about 6, about 5 to about 450, about 5 to about 400, about 5 to about 350, about 5 to about 300, about 5 to about 250, about 5 to about 200, about 5 to about 150, about 5 to about 100, about 5 to about 80, about 5 to about 60, about 5 to about 40, about 5 to about 20, about 5 to about 15, about 5 to about 10, about 5 to about 8, about 5 to about 6, about 10 to about 450, about 10 to about 400, about 10 to about 350, about 10 to about 300, about 10 to about 250, about 10 to about 200, about 10 to about 150, about 10 to about 100, about 10 to about 80, about 10 to about 60, about 10 to about 40, about 10 to about 20, about 10 to about 15, about 20 to about 450, about 20 to about 400, about 20 to about 350, about 20 to about 300, about 20 to about 250, about 20 to about 200, about 20 to about 150, about 20 to about 100, about 20 to about 80, about 20 to about 60, about 20 to about 40, about 30 to about 450, about 30 to about 400, about 30 to about 350, about 30 to about 300, about 30 to about 250, about 30 to about 200, about 30 to about 150, about 30 to about 100, about 30 to about 80, about 30 to about 60, about 30 to about 40, about 40 to about 450, about 40 to about 400, about 40 to about 350, about 40 to about 300, about 40 to about 250, about 40 to about 200, about 40 to about 150, about 40 to about 100, about 40 to about 80, about 40 to about 60, about 50 to about 450, about 50 to about 400, about 50 to about 350, about 50 to about 300, about 50 to about 250, about 50 to about 200, about 50 to about 150, about 50 to about 100, about 50 to about 80, about 50 to about 60, about 100 to about 450, about 100 to about 400, about 100 to about 350, about 100 to about 300, about 100 to about 250, about 100 to about 200, about 100 to about 150, about 200 to about 450, about 200 to about 400, about 200 to about 350, about 200 to about 300, about 200 to about 250, about 300 to about 450, about 300 to about 400, or 300 to about 350, but embodiments of the present disclosure are not limited thereto. When the number of carbon atoms in R or R' in Formula 1 is greater than 450, due to a large molecular weight of the hybrid copolymer, problems in stability and processability may occur while unnecessary side reactions may be involved in the synthesis of the hybrid copolymer.

In one embodiment, the hybrid copolymer may further include a known polyether polyol or a known polyester polyol to ensure the compatibility, but embodiments of the present disclosure are not limited thereto.

In one embodiment, the monomer for forming the polyether polyol may be selected from an alkylene oxide, a diol, a triol, or any combination thereof, and the monomer of the polyester polyol may be an organic acid including at least two carboxyl groups, but embodiments of the present disclosure are not limited thereto.

In one embodiment, the monomer for forming the polyether polyol may be selected from: an alkylene oxide selected from etylene oxide and propylene oxide; a dial selected from dipropylene glycol (DPG), mono-ethylene glycol (MEG) and propylene glycol (PG); a trial selected from glycerin and trimethylol propane (TMP); and any combination thereof, but embodiments of the present disclosure are not limited thereto.

In one embodiment, the polyether polyol may be obtained by addition polymerization between an active hydrogen atom-containing initiator and an alkylene oxide, such as propylene oxide or ethylene oxide, but embodiments of the present disclosure are not limited thereto. The initiator may be usually selected from: propylene glycol, dipropylene glycol, ethylene glycol, diethylene glycol, diethanol amine, bisphenol, and water, which have two functional groups; glycerine, trimethylol propane, triethanol amine, and haxanetriol, which have three functional groups; and any combination thereof, but embodiments of the present disclosure are not limited thereto.

In one embodiment, to enhance the hardness of the polyether polyol, an initiator prepared by copolymerizing a vinyl group-containing monomer, such as a styrene monomer or an acrylonitrile monomer, with the synthesized polyether polyol, or an initiator, such as pentaerythritol, ethylenediamine, toluenediamine, sorbitol, or sucrose, which has at least three functional groups, may be partially used at a ratio in a range of about 1% to about 30%, but embodiments of the present disclosure are not limited thereto.

In one embodiment, the monomer of the polyester polyol may include a multivalent organic acid including at least two carboxyl groups and about 2 to about 20 carbon atoms, and examples of the multivalent organic acid may be a higher fatty acid, a saturated fatty acid, or an unsaturated fatty acid, but embodiments of the present disclosure are not limited thereto. For example, the number of carbons atoms in the multivalent organic acid may be in a range of about 2 to about 20, about 2 to about 18, about 2 to about 16, about 2 to about 14, about 2 to about 12, about 2 to about 10, about 2 to about 8, about 2 to about 6, or about 2 to about 4, but embodiments of the present disclosure are not limited thereto.

In one embodiment, the multivalent organic acid including at least two carboxyl groups may be, for example, selected from adipic acid, oxalic acid, and succinic acid, but embodiments of the present disclosure are not limited thereto.

In one embodiment, the monomer of the polyester polyol may be selected from soybean oil, castor oil, citric acid, palmitic acid, myristic acid, oleic acid, stearic acid, linoleic acid, and linolenic acid, but embodiments of the present disclosure are not limited thereto.

In one embodiment, following the synthesis of the polyether polyol, an organic acid including at least two carboxylic acid, such as adipic acid or fatty acid, may be added thereto to induce esterification via dehydration condensation, so as to synthesize a copolymer in which ether-ester bonds coexist in one polymer. Here, the temperature at which the hybrid copolymer is synthesized via esterification may be room temperature or above room temperature, or may be in a range of about room temperature to about 250°C, about room temperature to about 200°C, about room temperature to about 150°C, about room temperature to about 100°C, about 50°C to about 250°C, about 50°C to about 200°C, about 50°C to about 150°C, about 50°C to about 100°C, about 100°C to about 250°C, about 100°C to about 200°C, about 100°C to about 150°C, about 150°C to about 250°C, about 150°C to about 200°C, or about 200°C to about 250°C, but embodiments of the present disclosure are not limited thereto.

In one embodiment, as polyurethane is formed by a reaction with a compound including an isocyanate group in the presence of a catalyst, a foam stabilizer, a foaming agent, and if needed, other flame retardants, fillers, lubricants, and cross-linking agents, the synthesized hybrid copolymer may form a hybrid foam. The polyurethane may be produced by synthesis between a material with an active hydrogen atom and a material with an isocyanate group (-NCO), but embodiments of the present disclosure are not limited thereto.

In one embodiment, the compound including the isocyanate group may be selected from aromatic, alicyclic, and aliphatic polyisocianates having at least two isocyanate groups, any combination thereof, and modified polyisocyanates obtained by modification performed on the aromatic, alicyclic, and aliphatic polyisocianates, but embodiments of the present disclosure are not limited thereto.

In one embodiment, the compound including the isocyanate group may be selected from: aromatic diisocyanate, such as methylene diphenyl diisocyanate (MDI), toluene diisocyanate (TDI), 2,4- or 2,6-tolylene diisocyanate, xylylene diisocyanate (XDI), paraphenylene diisocyanate, 1,5-naphthalene diisocyanate, or tolidine diisocyanate; aliphatic diisocyanate with an aromatic ring, such as α,α,α',α'-tetramethyi xylylene diisocyanate; aliphatic diisocyanate, such as methylene diisocyanate, propylene diisocyanate, lysine diisocyanate, 2,2,4- or 2,4,4-trimethyl hexamethylene diisocyanate, or 1,6-hexamethylene diisocyanate (HMDI); alicyclic diisocyanate, such as 1,4-cyclohexane diisocyanate, methylcyclohexane diisocyanate (TDI with hydrogen), 1-isocyanate-3-isocyanatemethyl-3,5,5-trimethylcyclohesane (IPDI), 4,4'-dicyclohexylmethane diisocyanate, or isopropylidenedicyclohexyl-4,4'-diisocyanate; and any combination thereof, but embodiments of the present disclosure are not limited thereto.

In one embodiment, the amount of the polyisocyanate compound being used may be in a range of about 30 to about 130, about 40 to about 120, or about 60 to about 100, and more preferably, about 50 to about 110, in terms of an isocyanate index, but embodiments of the present disclosure are not limited thereto. The isocyanate index refers to a numerical value which is about 100 times the number of the isocyanate group(s) with respect to the total of all the active hydrogen-rich water molecules including polyols, cross-linking agents, water, or the like.

In one embodiment, the catalyst may include any catalyst that promotes urethanization. For example, the catalyst may be selected from: tertiary amines, such as triethylenediamine, bis(2-dimethylaminoethyl)ether, and N,N,N',N'-tetramethylhexa methylenediamine; metal salts of carboxylic acids, such as potassium acetate and 2-ethylhexane potassium; organometallic compounds, such as stannous octoate, and dibutyl tin dilaurate; and any combination thereof, but embodiments of the present disclosure are not limited thereto.

In one embodiment, the foam stabilizer may be used without limitation as being commonly used in the production of a polyurethane form, and examples thereof may include a silicon-based foam stabilizer and a fluorine-based foam stabilizer. However, embodiments of the present disclosure are not limited thereto.

In one embodiment, the foam stabilizer may be preferably a silicon-based foam stabilizer, and the silicon content of the foam stabilizer may be in a range of about 10 parts to about 50 parts by weight, more preferably, about 30 parts to about 50 parts by weight, based on 100 parts by weigh of the foam stabilizer. However, embodiments of the present disclosure are not limited thereto. The silicon content is a ratio of a polysiloxane chain to the foam stabilizer, and the remainder is a polyoxyalkylene chain. In addition, the content of an oxyethylene group in the polyoxyalkylene chain may be in a range of about 70 parts to about 100 parts, more preferably, about 90 parts to about 100 parts by weight, based on 100 parts by weight of the polyoxyalkylene chain. However, embodiments of the present disclosure are not limited thereto.

In one embodiment, the foaming agent may be selected from fluorinated hydrocarbon, water, and inert gas, and any combination thereof, and the inert gas may include air, nitrogen gas, and carbon dioxide gas. The amount of the foaming agent being used is not particularly limited, but when water is used, the amount of the foaming agent may be about 10 parts by weight or less or in a range of about 0.5 parts to about 4 parts by weight, based on 100 parts by weight of the polyol mixture. However, embodiments of the present disclosure are not limited thereto.

In one embodiment, an additive may be further added in the process of forming the polyurethane. The additive may be, for example, selected from: a filler, such as potassium carbonate or barium sulfate; a surfactant, such as an emulsifier; an antiaging agent, such as antioxidant or a UV absorber; a flame retardant; a plasticizer; a coloring agent; an antifungal agent; a defoamer; a dispersant; anti-tarnish agent; and any combination thereof, but embodiments of the present disclosure are not limited thereto.

In one embodiment, the amount of the foam stabilizer may be in a range of about 0.01 parts to about 5 parts by weight, more preferably, about 0.1 parts to about 2 pars by weight, based on 100 parts by weight of the polyol (wherein, a cross-linking agent is not included in the polyol), but embodiments of the present disclosure are not limited thereto.

In one embodiment, a dial including DPG as an initiator may be subjected to addition polymerization with PO, and accordingly, a polyether polyol having a molecular weight in a range of about 100 to about 8,000 may be primarily formed. Then, an organic acid, such as adipic acid, may be added to the polymerized polyether polyol, and via dehydration condensation, the resulting compound may be esterified, thereby forming a copolymer having a hybrid structure of a polyester polyol and a polyether polyol that are copolymerized. To the copolymer formed therefrom, a pigment, a vulcanizing agent, a foaming agent, other additives, and water may be added and mixed. The mixture may be then stirred with isocyanate to foam, followed by being injected to a mold to solidify. Afterwards, the foam molded as being subjected to a heating process in a heating furnace and to vulcanization may be cut, dried, and surface-treated, thereby preparing a hybrid foam, but embodiments of the present disclosure are not limited thereto.

In one embodiment, the molecular weight of the hybrid copolymer may be in a range of about 100 to about 20,000, about 100 to about 18,000, about 100 to about 16,000, about 100 to about 14,000, about 100 to about 12,000, about 100 to about 10,000, about 100 to about 9,000, about 100 to about 8,000, about 100 to about 7,000, about 100 to about 6,000, about 100 to about 5,000, about 100 to about 4,000, about 100 to about 3,000, about 100 to about 2,000, about 500 to about 20,000, about 500 to about 18,000, about 500 to about 16,000, about 500 to about 14,000, about 500 to about 12,000, about 500 to about 10,000, about 500 to about 9,000, about 500 to about 8,000, about 500 to about 7,000, about 500 to about 6,000, about 500 to about 5,000, about 500 to about 4,000, about 500 to about 3,000, about 500 to about 2,000, about 1,000 to about 20,000, about 1,000 to about 18,000, about 1,000 to about 16,000, about 1,000 to about 14,000, about 1,000 to about 12,000, about 1,000 to about 10,000, about 1,000 to about 9,000, about 1,000 to about 8,000, about 1,000 to about 7,000, about 1,000 to about 6,000, about 1,000 to about 5,000, about 1,000 to about 4,000, about 1,000 to about 3,000, about 1,000 to about 2,000, about 2,000 to about 20,000, about 2,000 to about 18,000, about 2,000 to about 16,000, about 2,000 to about 14,000, about 2,000 to about 12,000, about 2,000 to about 10,000, about 2,000 to about 9,000, about 2,000 to about 8,000, about 2,000 to about 7,000, about 2,000 to about 6,000, about 2,000 to about 5,000, about 2,000 to about 4,000, about 2,000 to about 3,000, about 3,000 to about 20,000, about 3,000 to about 18,000, about 3,000 to about 16,000, about 3,000 to about 14,000, about 3,000 to about 12,000, about 3,000 to about 10,000, about 3,000 to about 9,000, about 3,000 to about 8,000, about 3,000 to about 7,000, about 3,000 to about 6,000, about 3,000 to about 5,000, about 3,000 to about 4,000, about 4,000 to about 20,000, about 4,000 to about 18,000, about 4,000 to about 16,000, about 4,000 to about 14,000, about 4,000 to about 12,000, about 4,000 to about 10,000, about 4,000 to about 9,000, about 4,000 to about 8,000, about 4,000 to about 7,000, about 4,000 to about 6,000, about 4,000 to about 5,000, about 5,000 to about 20,000, about 5,000 to about 18,000, about 5,000 to about 16,000, about 5,000 to about 14,000, about 5,000 to about 12,000, about 5,000 to about 10,000, about 5,000 to about 9,000, about 5,000 to about 8,000, about 5,000 to about 7,000, about 5,000 to about 6,000, about 6,000 to about 20,000, about 6,000 to about 18,000, about 6,000 to about 16,000, about 6,000 to about 14,000, about 6,000 to about 12,000, about 6,000 to about 10,000, about 6,000 to about 9,000, about 6,000 to about 8,000, about 6,000 to about 7,000, about 7,000 to about 20,000, about 7,000 to about 18,000, about 7,000 to about 16,000, about 7,000 to about 14,000, about 7,000 to about 12,000, about 7,000 to about 10,000, about 7,000 to about 9,000, about 7,000 to about 8,000, about 8,000 to about 10,000, about 8,000 to about 9,000, about 2,500 to about 3,500, about 3,500 to about 4,500, 4,500 to about 5,500, or about 5,500 to about 6,500. When the molecular weight of the hybrid copolymer is less than 1,000, the elasticity of the foam including the hybrid copolymer may be reduced while the hardness thereof may be increased, resulting in a reduced sense of touch. When the molecular weight of the hybrid copolymer is greater than 20,000, the processing of the foam including the hybrid copolymer may be difficult, and the foam may be so soft that the durability of the foam may be reduced.

In one embodiment, the hybrid foam may include open pores, but embodiments of the present disclosure are not limited thereto.

In one embodiment, the number of number of pores per inch (ppi) of the open pores in the hybrid foam may be in a range of about 70 ppi to about 120 ppi, about 70 ppi to about 110 ppi, about 70 ppi to about 100 ppi, about 80 ppi to about 120 ppi, about 80 ppi to about 110 ppi, about 80 ppi to about 100 ppi, about 90 ppi to about 120 ppi, about 90 ppi to about 110 ppi, or about 90 ppi to about 100 ppi, but embodiments of the present disclosure are not limited thereto. When the number of the open pores in the hybrid foam is less than about 70 ppi, the elasticity of the foam may be reduced, resulting in poor usability, and the fluidity of the cosmetic composition may be difficult to control. When the number of the open pores in the hybrid foam is greater than about 120 ppi, the durability and usability of the contents may be degraded.

In one embodiment, the size of the open pores in the hybrid foam may be in a range of about 0.01 mm to about 0.35 mm, about 0.05 mm to about 0.35 mm, about 0.10 mm to about 0.35 mm, about 0.15 mm to about 0.35 mm, about 0.20 mm to about 0.35 mm, about 0.25 mm to about 0.35 mm, or about 0.30 mm to about 0.35 mm, but embodiments of the present disclosure are not limited thereto. When the size of the open pores in the hybrid foam is smaller than the range above, the impregnation of the hybrid foam may be difficult, and in addition, the usage efficiency of hybrid foam may be small compared with the amount of the contents filled therein. When the size of the open pores in the hybrid foam is greater than the range above, the usage efficiency of hybrid foam may be good compared with the amount of the contents filled therein. However, when the pore size is too large, the stability of the contents of the hybrid foam is not good so that the strength required for collecting the cosmetic composition is small, resulting in easy discharge of the contents upon a weak impact applied thereto. That is, there may be a problem with poor discharge control ability of the cosmetic composition.

In one embodiment, by using an Asker hardness tester type F, the hardness of the hybrid foam may be measured in a range of about 10 to about 70, about 10 to about 60, about 10 to about 50, about 10 to about 40, about 10 to about 30, or about 10 to about 20, but embodiments of the present disclosure are not limited thereto.

In one embodiment, the cosmetic composition impregnated in the hybrid foam may have the viscosity in a range of about 4,000 cps to about 20,000 cps, about 5,000 cps to about 20,000 cps, about 6,000 cps to about 20,000 cps, about 7,000 cps to about 20,000 cps, about 8,000 cps to about 20,000 cps, about 9,000 cps to about 20,000 cps, about 10,000 cps to about 20,000 cps, about 11,000 cps to about 20,000 cps, about 12,000 cps to about 20,000 cps, about 13,000 cps to about 20,000 cps, about 14,000 cps to about 20,000 cps, about 15,000 cps to about 20,000 cps, about 16,000 cps to about 20,000 cps, about 17,000 cps to about 20,000 cps, about 18,000 cps to about 20,000 cps, or about 19,000 cps to about 20,000 cps, but embodiments of the present disclosure are not limited thereto. When the viscosity of the cosmetic composition impregnated in the hybrid foam is less than about 4,000 cps, the cosmetic composition is not proper for use since the bulk-state contents thereof before impregnation are quickly likely to be separated. When the viscosity of the cosmetic composition impregnated in the hybrid foam is greater than about 20,000 cps, which is too high, there may be a difficulty in impregnation and a problem that the feeling of thin and light, which is an advantage of a cushion, may be reduced.

In one embodiment, the cosmetic composition impregnated in the hybrid foam may be a makeup base, a BB cream, a skin cover, a makeup foundation, a makeup primer, a blusher, a lipstick, a lip-gloss, a face powder, a lip liner, an eyebrow pencil, an eye shadow, a compact powder, a twin cake compact powder, a concealer, or a sunscreen, but embodiments of the present disclosure are not limited thereto.

Hereinafter, the present disclosure will be described in more detail with reference to Examples. However, Examples shown and described herein are illustrative examples of embodiments and are not intended to otherwise limit the scope of embodiments in anyway.

### MODE OF DISCLOSURE

### [Examples]

### 1. Specification and preparation of control group and test group

### (1) Specification of control group and test group

The existing polyether polyol foam and the existing polyester polyol foam were each used as a control group, and a hybrid foam used as a test group.

### (2) Preparation of polyether polyol foam (control group)

50 parts by weight (molecular weight of 6,000) of polyether polyolA, 50 parts by weight (molecular weight of 3,000) of copolymerized polyether polyol B, 1.2 parts by weight of cell opener, 0.8 parts by weight of silicon surfactant, 1.0 parts by weight of diethanolamine, 3.9 parts by weight of water, and 0.44 parts by weight of catalyst were mixed for preparation, and the mixed product was called modified methylene diphenyl diisocyanate (MDI, NCO%=29.8, Product Name: CG-3,000 (Kumho Chemical). MDI was used at an index of 100 for a reaction in a low-pressure mixing machine. All the components were contacts at a temperature of about 25°C. The resulting reaction mixture was then dispensed into a mold at a temperature of 60°C, and cured at room temperature for 5 minutes. After removing from the mold, the foam specimen was sufficiently compressed to open the cell areas.

### (3) Preparation of polyester polyol foam (control group)

50 parts by weight (molecular weight of 3,000) of polyester polyol A, 50 parts by weight (molecular weight of 1,000) of polyester polyol B, 1.2 parts by weight of cell opener, 0.8 parts by weight of silicon surfactant, 1.0 parts by weight of diethanolamine, 3.9 parts by weight of water, and 0.47 parts by weight of catalyst were 0.47 mixed for preparation, and the mixed product was called MDI (NCO%=29.8, Product Name: CG-3,000 (Kumho Chemical). MDI was used at an index of 100 for a reaction in a low-pressure mixing machine. All the components were contacts at a temperature of about 25°C. The resulting reaction mixture was then dispensed into a mold at a temperature of 60°C, and cured at room temperature for 5 minutes. After removing from the mold, the foam specimen was sufficiently compressed to open the cell areas.

### (4) Preparation of hybrid foam (test group)

50 parts by weight (molecular weight of 3,500) of hybrid copolymer of polyether polyol and polyester polyol (molecular weight of polyether polyol in the hybrid polyol copolymer was 3,000), 1.2 parts by weight of cell opener, 0.8 parts by weight of silicon surfactant, 1.0 parts by weight of diethanolamine, 3.9 parts by weight of water, and 0.44 parts by weight of catalyst were mixed for preparation, and the mixed product was called MDI (NCO%=29.8, Product Name: CG-3,000 (Kumho Chemical). MDI was used at an index of 100 for a reaction in a low-pressure mixing machine. All the components were contacts at a temperature of about 25°C. The resulting reaction mixture was then dispensed into a mold at a temperature of 60°C, and cured at room temperature for 5 minutes. After removing from the mold, the foam specimen was sufficiently compressed to open the cell areas. The hybrid foam prepared therefrom had the Asker hardness in a range of about 10 to about 70, and the number of pores in the hybrid foam was in a range of about 70 ppi to about 120 ppi.

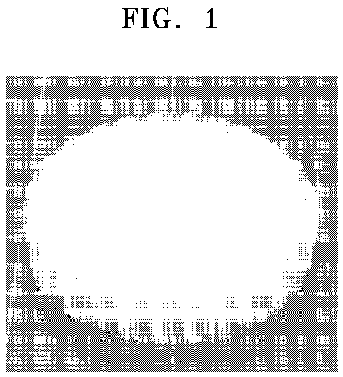

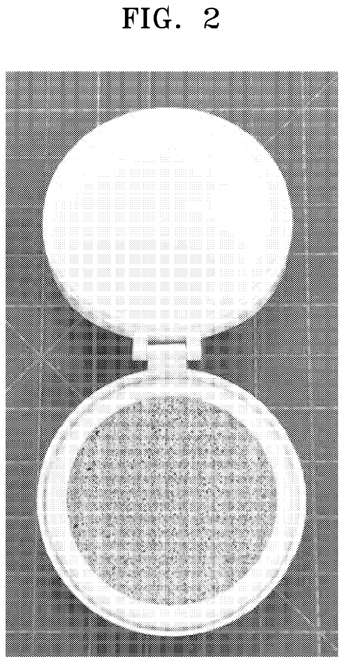

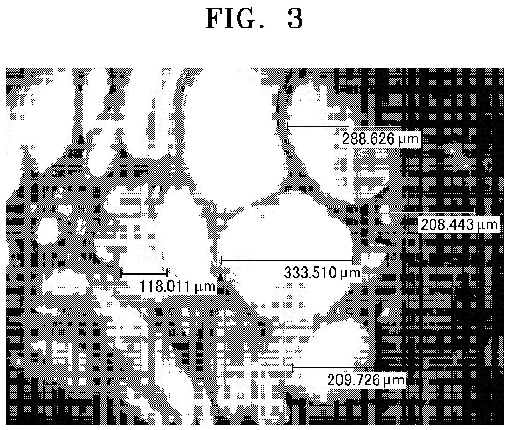

FIG. 1 is a photograph of a hybrid foam prepared according to an embodiment; FIG. 2 is a photograph of a hybrid foam impregnated with the cosmetic composition of the present disclosure; and FIG. 3 is an optical microscopic photograph of a hybrid foam prepared according to an embodiment.

As shown in FIG. 1, the prepared hybrid foam was cut into 48 mm X 8 mm. As shown in FIG. 3, the prepared hybrid foam included open pores, wherein the inner size of the pores was measured to be in a range of about 0.1 mm to 0.35 mm (optical microscope, OLYMPUS BX51, 10x/0.3 magnification).

### 2. Test on deformation stability against chemical resistance

When the foam is impregnated with the cosmetic composition, the foam may expand in volume over time. Thus, in order to embed the foam in a container of a certain size, such a volume expansion phenomenon must be considered. Since the volume expansion appears remarkably in the case of the polyether polyol foam, a test was carried out to confirm whether the hybrid foam of the present disclosure can solve this problem.

The polyether polyol foam prepared as the control group and the hybrid foam prepared as the test group were each impregnated with ingredients of the cosmetic composition, such as ester oil, polar organic sunscreen, and volatile silicone oil, and then, were maintained at a temperature of 80°C for 20 minutes. Afterwards, the volume of the impregnated foam was compared with that of the foam before the impregnation to confirm the volume expansion upon the impregnation, and the results are shown in [Table 1].

The ester oil used herein was cetyl ethylhexanoate, diisostearyl malate, butylene glycol dicaprylate/dicaprate, dicaprylyl carbonate, or neopentyl glycol diheptanoate. The polar organic sunscreen used herein was representatively ethylhexyl methoxycinnamate, ethylhexyl salicylate, or isoamyl-P-methoxycinnamate. The volatile silicone oil used herein was cyclohexasiloxane, cyclopentasiloxane, or cyclomethicone.

**[Table 1]**

| | Polyether polyol foam (control group) | Hybrid foam (test group) |
|---|---|---|
| Ester oil | 4.5% | 2.0% |
| Polar organic sunscreen | 8.0% | 5.0% |
| Volatile silicone oil | 0% | 0% |

As shown in [Table 1], as a result of performing impregnation with ester oil, polar organic sunscreen, and volatile silicone oil, the hybrid foam of the present disclosure showed a volume expansion rate that was about half the volume expansion rate of the existing polyether polyol foam. Therefore, it was confirmed that the hybrid foam of the present disclosure was excellent in the stability against the chemical resistance, compared with the existing polyether polyol foam.

### 3. Test on stability against hydrolysis

The cosmetic composition was used in various formulations, and thus, the foam to be impregnated therewith must be formed of a material resistant to both oil and moisture. The existing polyester polyol foam was difficult to use in cosmetics due to hydrolytic properties. Thus, a test was carried out to confirm whether the hybrid foam of the present disclosure was able to solve the problem of hydrolysis.

To evaluate the hydrolytic properties of the foam, the polyester polyol foam and the hybrid foam were each impregnated with 10 wt% hydrochloric acid and 10 wt% nitric acid at room temperature for 7 days, and then, the collapse of each foam was observed. To proceed the test under accelerated conditions, acid catalysts, i.e., hydrochloric acid and nitric acid, were used at an amount of 10 wt%.

**[Table 2]**

| | Polyester polyol foam (control group) | Hybrid foam (test group) |
|---|---|---|
| 10 wt% hydrochloric acid | X | ○ |
| 10 wt% nitric acid | X | ○ |

| | | |
|---|---|---|
| 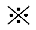 Evaluation criteria (○: collapse, X: no collapse) | | |

As shown in [Table 2], it was confirmed that the hybrid foam was stable against hydrolysis, whereas the polyester polyol foam showed a collapse.

### 4. Test on durability

A cosmetic composition used in the durability test was prepared according to ingredients shown in [Table 3].

**[Table 3]**

| | Raw material | Content (%) |
|---|---|---|
| Oil-phase ingredient | Cyclopentasiloxane | 14.5 |
| | Ethylhexyl methoxycinnamate | 7.5 |
| | Ethylhexyl salicylate | 4.0 |
| | Caprylic/capric triglyceride | 5.0 |
| | PEG-10 dimethicone | 3.0 |
| | Diphenylsiloxy phenyl trimethicone | 2.0 |
| | Dimethicone | 2.0 |
| | Sorbitan isostearate | 1.0 |
| Thickener | Disteardimonium hectorite | Suitable amount |
| Pigment | Titanium dioxide | 4.0 |
| | Titanium dioxide Aluminum hydroxide Stearic acid | 9.0 |
| | Yellow iron oxide | 0.8 |
| | Red iron oxide | 0.2 |
| | Black iron oxide | 0.1 |
| | Mica | 4.0 |

| | | |
|---|---|---|
| Water-phase ingredient | Purified water | To 100 |
| | Glycerin | 5.0 |
| | Salt | 1.0 |

A cosmetic composition including the ingredients shown in [Table 3] was prepared as follows: the oil-phase ingredients were mixed with the thickener first, the pigment was added to the oil-phase ingredients including the thickener, and the mixture was homogeneously dispersed with a homomixer, and then, water-phase ingredient was slowly added thereto, followed by being homogeneously dispersed with a homomixer for emulsification, thereby preparing a W/O type foundation with low viscosity. The W/O type refers to a form in which water is contained in the inner surface while oil is wrapped around the outer surface.

When the hybrid foam of the present disclosure is actually used as a part of cosmetic products, a spreading puff is used, and thus, continuous pressure and friction are applied to the hybrid foam. The foam thus damaged is unsuitable for the purpose, and must have sufficient durability. Therefore, a test was carried out to confirm whether the hybrid foam of the present disclosure had better durability than that of the existing foam.

12 g of the cosmetic composition prepared by using the ingredients of [Table 3] was added and impregnated in each of the polyether foam, the polyester foam, and the hybrid foam, and then, each foam impregnated with the cosmetic composition was repeatedly pressed 10 times with the same pressure. Then, the resulting foams were stored in a constant temperature bath (25°C) for 24 hours, and such a process of impregnation, compression, and storage was repeated every day for 4 weeks. As shown in [Table 4], each form was evaluated by visual observation.

**[Table 4]**

| | Polyether polyol foam (control group) | Polyester polyol foam (control group) | Hybrid foam (test group) |
|---|---|---|---|
| Day 1 | ○ | ○ | ○ |
| Day 3 | ○ | ○ | ○ |
| Day 5 | ○ | ○ | ○ |
| Day 7 | ○ | ○ | ○ |
| Week 2 | ○ | ○ | ○ |
| Week 3 | ○ | ○ | ○ |
| Week 4 | Δ | Δ | ○ |

| | | | |
|---|---|---|---|
| 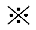 Stability evaluation criteria (○: Satisfactory, Δ: Normal, X: Poor) | | | |

As shown in [Table 4], the continuous and repetitive pressure was applied to each of the polyether polyol foam, the polyester polyol foam, and the hybrid foam. The immersed cosmetic composition was W/O type, and it was confirmed that the hybrid foam of the present disclosure showed the best durability during the 4-week test.

Although the one or more embodiments of the present disclosure have been disclosed for illustrative purposes, those skilled in the art will appreciate that various modifications and variations are possible, without departing from the scope and spirit of the appended claims. Therefore, it will be understood that the one or more embodiments described above are for illustrative in all aspects, and are construed as not being definitely limited. For example, each component described as a single entity may be distributed and implemented, and components described as being distributed may also be implemented in a combined form.

While the present disclosure been particularly shown and described with reference to exemplary embodiments thereof, it will be understood by those skilled in the art that various changes in form and details may be made therein without departing from the spirit and scope of embodiments as defined by the appended claims. The exemplary embodiments should be considered in descriptive sense only and not for purposes of limitation. Therefore, the scope of embodiments is defined not by the detailed description of embodiments but by the appended claims, and all differences within the scope will be construed as being included in the inventive concept.

## Claims

1. A hybrid foam for use in impregnating a cosmetic composition, comprising a hybrid copolymer of a polyether polyol and a polyester polyol.

2. The hybrid foam of claim 1, wherein the hybrid copolymer comprises a copolymer formed by copolymerizing X and Y, wherein X indicates an ether structure-containing polyol or a monomer or oligomer for forming the polyether polyol, and Y indicates an ester structure-containing polyol or a monomer or oligomer for forming the polyester polyol.

3. The hybrid foam of claim 2, wherein the copolymer formed by copolymerizing X and Y is represented by (XY)ₙX, wherein n is a number in a range of 1 to 200.

4. The hybrid foam of claim 2, wherein the monomer for forming the polyether polyol is selected from the group consisting of an alkylene oxide, a diol, a triol, and any combination thereof, and the monomer for forming the polyester polyol comprises an organic acid containing at least two carboxyl groups.

5. The hybrid foam of claim 1, wherein the hybrid copolymer has a molecular weight in a range of 100 to 8,000.

6. The hybrid foam of claim 1, wherein the hybrid foam comprises open pores.

7. The hybrid foam of claim 6, wherein a number of pores per inch (ppi) of the open pores is in a range of about 70 ppi to about 120 ppi, and the open pores have a size in a range of about 0.01 mm to about 0.35 mm.

8. The hybrid foam of claim 1, wherein the hybrid foam has a hardness in a range of about 10 to about 70 when measured with an Asker hardness tester type F.

9. A cosmetic kit comprising:
a cosmetic composition; and
a hybrid foam comprising a hybrid copolymer of a polyether polyol and a polyester polyol,
wherein the hybrid foam is impregnated with the cosmetic composition.

10. The cosmetic kit of claim 9, further comprising a spreading applicator.

11. The cosmetic kit of claim 9, further comprising a container for accommodating the hybrid foam.

12. The cosmetic kit of claim 9, further comprising an inner container for accommodating the hybrid foam and an outer container containing the inner container.

13. The cosmetic kit of claim 9, wherein the hybrid copolymer comprises a copolymer formed by copolymerizing X and Y, wherein X indicates an ether structure-containing polyol or a monomer or oligomer for forming the polyether polyol, and Y indicates an ester structure-containing polyol or a monomer or oligomer for forming the polyester polyol.

14. The cosmetic kit of claim 13, wherein the copolymer formed by copolymerizing X and Y is represented by (XY)ₙX, wherein n is a number in a range of 1 to 200.

15. The cosmetic kit of claim 13, the monomer for forming the polyether polyol is selected from the group consisting of an alkylene oxide, a diol, a triol, and any combination thereof, and the monomer of the polyester polyol comprises an organic acid containing at least two carboxyl groups.

16. The cosmetic kit of claim 9, wherein the hybrid copolymer has a molecular weight in a range of 100 to 8,000.

17. The cosmetic kit of claim 9, wherein the hybrid foam comprises open pores.

18. The cosmetic kit of claim 17, wherein a number of pores per inch (ppi) of the pores is in a range of about 70 ppi to about 120 ppi, and the pores have a size in a range of about 0.01 mm to about 0.35 mm.

19. The cosmetic kit of claim 9, wherein the hybrid foam has a hardness in a range of about 10 to about 70 when measured with an Asker hardness tester type F.

20. The cosmetic kit of claim 9, wherein the cosmetic composition has a viscosity in a range of about 4,000 cps to about 20,000 cps.

21. The cosmetic kit of claim 9, wherein the cosmetic composition comprises a makeup base, a BB cream, a skin cover, a makeup foundation, a makeup primer, a blusher, a lipstick, a lip-gloss, a face powder, a lip liner, an eyebrow pencil, an eye shadow, a compact powder, a twin cake compact powder, a concealer, or a sunscreen.
